# EUROPÄISCHE PATENTSCHRIFT

(11) **EP 1 153 413 B1**
(45) Veröffentlichungstag und Bekanntmachung des Hinweises auf die Patenterteilung: **14.11.2007**
(21) Anmeldenummer: 00907589.6
(22) Anmeldetag: 18.02.2000
(51) Int. Cl.: H01J 47/02, G01T 1/185

(54) **IONISATIONSKAMMER FÜR IONENSTRAHLEN**
IONIZATION CHAMBER FOR ION BEAMS
CHAMBRE D'IONISATION POUR FAISCEAUX IONIQUES

(30) Priorität: 19.02.1999 DE 19907207
(43) Veröffentlichungstag der Anmeldung: 14.11.2001
(73) Patentinhaber: Gesellschaft für Schwerionenforschung mbH, 64291 Darmstadt (DE)
(72) Erfinder: STELZER, Herbert, D-64291 Darmstadt (DE); VOSS, Bernd, D-64291 Darmstadt (DE)
(74) Vertreter: Boeters, Hans Dietrich
(86) Internationale Anmeldenummer: PCT/EP2000/001390
(87) Internationale Veröffentlichungsnummer: WO 2000/049639

(56) Entgegenhaltungen:
- US-A- 4 686 368
- US-A- 5 120 967
- US-A- 5 326 976
- US-A- 5 508 526
- KRAFT G ET AL: "Heavy ion therapy at GSI" NUCLEAR INSTRUMENTS & METHODS IN PHYSICS RESEARCH, SECTION - A: ACCELERATORS, SPECTROMETERS, DETECTORS AND ASSOCIATED EQUIPMENT,NL,NORTH-HOLLAND PUBLISHING COMPANY. AMSTERDAM, Bd. A367, Nr. 1, 11. Dezember 1995 (1995-12-11), Seiten 66-70, XP004006159 ISSN: 0168-9002

## Beschreibung

Die Erfindung betrifft eine Ionisationskammer für Ionenstrahlen gemäß dem Gattungsbegriff des Anspruchs 1.

Derartige Ionisationskammern sind auch als Ionenzählrohre aus dem Stand der Technik bekannt. Das Kammergehäuse wird üblicherweise aus einem Rohr gebildet, wobei einer der beiden Endabschnitte des Rohres als Strahleinlaßfenster und das andere Ende des Rohres als Strahlauslaßfenster dient. Das Rohr ist mit einem Zählgas unter vermindertem Druck gefüllt und weist eine zylindrische Hochspannungskathode auf, die koaxial und isoliert von der Rohrwandung in dem Zählrohr liegt. Im Zentrum des Rohres befindet sich eine zylindrische Hochspannungsanode, die von der Hochspannungskathode und dem umgebenden Rohr isoliert ist. Zwischen Hochspannungskathode und Hochspannungsanode wird zum Betrieb der Ionisationskammer eine Spannung angelegt und der Strom zwischen Kathode und Anode gemessen. Werden geladene Teilchen wie Ionen durch die Ionisationskammer geleitet oder von der-Ionisationskammer eingefangen, so erhöht sich der Strom zwischen Kathode und Anode abhängig von der Zahl der Ionen, die die Ionisationskammer passieren. Komplexere zylindrische Ionisationskammern weisen eine Vielzahl axial ausgerichteter Anoden auf, um beispielsweise den Weg eines geladenen Teilchens oder Ions durch eine rohrförmige Ionisationskammer mit über den Querschnitt in Axialrichtung verteilten Anoden zu messen.

Derartige zylindrische Ionisationskammern haben den Nachteil einer großen axialen Erstreckung und eines relativ komplexen Aufbaus der Zählanoden. Darüber hinaus ist der Platzbedarf derartiger Ionisationskammern in Richtung der Ausbreitung eines Strahls relativ groß. Der verfügbare Platz am Strahlaustritt in einem Behandlungsraum vor Patienten ist jedoch eng begrenzt. Darüber hinaus muß bei derartigen Therapiesystemen, bei denen ein Ionenstrahl über die Gesamtausdehnung eines Tumorgewebes gescannt wird, eine Ionisationskammer zur Verfügung stehen, die in Breiten- und Längenausdehnung bisher nicht bekannte Ausmaße annimmt. Generell sind alle Messungen im Strahl vor dem Patienten im Transmissionsmodus durchzuführen. Dabei ist eine Verschlechterung der Strahlqualität, wie etwa durch Projektilfragmentation und Winkelstreuung der Strahlteilchen, unbedingt zu vermeiden.

Aufgabe der Erfindung ist es deshalb, eine Ionisationskammer für Ionenstrahlen anzugeben, der die Nachteile bisheriger Ionisationskammern überwindet, für die Überwachung und Steuerung einer Patientenbestrahlung im Rahmen einer Tumortherapie mit schweren Ionen, die mit hoher Energie in einem Bleistiftstrahl konzentriert sind, geeignet ist, in Strahlrichtung geringe Detektorabmessungen aufweist, eine hohe Sicherheit, insbesondere im Hinblick auf Plasma- und Funkenbildung ermöglicht, und im medizinischen Bereich einsetzbar ist.

Diese Aufgabe wird mit dem Gegenstand des Anspruchs 1 gelöst.

Dazu besteht die Ionisationskammer für Ionenstrahlen aus einem Kammergehäuse, einem Strahleneinlaßfenster und einem Strahlenauslaßfenster, einem mit Zählgas gefüllten Kammervolumen und einer Hochspannungsanode und einer Hochspannungskathode. Die Ionisationskammer ist flach und sandwichartig aus plattenförmigen großflächen Strukturen dieser Komponenten aufgebaut, die orthogonal zur Ionenstrahlachse ausgerichtet sind. Dabei ist eine zentral angeordnete großflächige orthogonal ausgerichtete plattenförmige Zählanode von einer großflächigen plattenförmigen Hochspannungskathode aus zwei parallelen Kathodenplatten beidseitig umgeben. Das Kammergehäuse besteht aus einem Gehäuserahmen, der ein Ionisationskammervolumen nahezu quadratisch umrahmt, und auf dem das Strahleinlaß- und Strahlauslaßfenster gasdicht angebracht sind. Eine derartige Vorrichtung hat den Vorteil einer erhöhten Wartungsfreundlichkeit, da die Plattenkonstruktion durch einfaches Abtragen oder Abnehmen unterschiedlicher Plattenstrukturen aus dem Gehäuserahmen entnommen und gewechselt werden kann. Die Platten können leicht ausgetauscht werden und in entsprechender Stückzahl auf Vorrat gehalten werden. Die Plattenstruktur ermöglicht auch eine Massenherstellung von Einzelteilen und fertigen Ionisationskammern.

In einer bevorzugten Ausführungsform der Erfindung ist das Zählgas ein Gasgemisch aus Argon oder Krypton und Kohlendioxid, das vorzugsweise ein Gasvolumen-Mischungsverhältnis von 4:1, das an den Ionenstrahl im Hinblick auf Energie und Intensität angepaßt ist, aufweist und in die Ionisationskammer eingeleitet wird. Ein derartig zusammengesetztes Zählgas hat gegenüber den üblichen luftgefüllten zylindrischen Ionisationskammern den Vorteil einer höheren Reproduzierbarkeit der Messungen, da hier die Luftfeuchte die Sensitivität der Ionisationskammer nicht beeinflußt. Mit einem derartigen Zählgas wird ein gutes Signal-/Rauschverhältnis sichergestellt und ein hoher dynamischer Bereich in den Teilchenraten zur Verfügung gestellt. Darüber hinaus wird mit dem bevorzugten Zählgas eine ausreichende Durchschlagfestigkeit sichergestellt.

Ein derartiges Zählgas ist vorzugsweise von höchster Reinheit, da die Signalempfindlichkeit insbesondere in ihrer Höhe und ihrem Verlauf durch Verunreinigungen negativ beeinflußt wird. Vorzugsweise werden darüber hinaus innerhalb des Kammervolumens für die einzelnen Plattenelemente und sonstige Stütz- und Isolationselemente, sowie für Hilfsaggregate und Sensoren nicht gasende Materialien eingesetzt oder gasende Elemente und Bauteile in Epoxidharz eingegossen.

In einer weiteren bevorzugten Ausführungsform weist die Ionisationskammer Sensoren auf, die in gasdicht abgeschlossenen Durchgangsöffnungen im Gehäuserahmen montiert sind und den Zählgasdruck und die Zählgastemperatur messen. Die Ionisationskammer wird mit leichtem Überdruck gegenüber der umgebenden Luft betrieben, womit ein Eindringen von Fremdgasen vorteilhaft erschwert wird. Dazu wird die Größe des Gasrückflusses aus den Kammern durch eine Sensorik im Zählgasablaßbereich bzw. im Auslaß überwacht. Durch die Messung von Gasdruck und Gastemperatur kann vorteilhafterweise die Gasdichte, die direkt in die Bestimmung der Ionenstrahl-Teilchenzahl eingeht, überwacht und nötigenfalls konstant gehalten werden.

Das Strahleinlaßfenster und das Strahlauslaßfenster, die im wesentlichen quadratisch sind, bestehen vorzugsweise aus strahlungsbeständigen nichtpolarisierbaren Kunststoffolien. Diese sind an metallischen, plattenförmigen Rahmen befestigt, welche ihrerseits mittels O-Ring-Dichtungen im Gehäuserahmen das Ionisations-kammervolumen gasdicht zu dem Strahleneinlaßfenster und dem Strahlenauslaßfenster abschließen. Dieser gasdichte Aufbau hält Verunreinigungen von dem Zählgas fern und der Gasaustausch des Kammervolumens mit der Umgebung infolge von Diffusion wird auch bei Nichtbetrieb der Ionisationskammern minimiert.

Das Strahleinlaßfenster und das Strahlauslaßfenster weisen vorzugsweise Polyimid- oder Polyesterfolien auf, womit der Vorteil verbunden ist, daß ausschließlich strahlungsbeständige und nicht polarisierbare Materialien dem Ionenstrahl ausgesetzt werden und somit die Rückwirkung auf den Ionenstrahl und die Ionenstrahlintensität minimiert wird.

In einer weiteren bevorzugten Ausführungsform der Erfindung sind das Strahleinlaßfenster und das Strahlauslaßfenster zum Ionisationskammervolumen hin metallisiert. Mit einer derartigen Metallisierung des Strahleinlaßfensters und des Strahlauslaßfensters wird eine Aufladung der Fenster und damit eine Verfälschung der Meßwerte verhindert, da Ladungen unmittelbar über die Metallisierung der Fenster und über den Rahmen der Fenster an das Ionisationskammergehäuse abgeleitet werden können. Das Ionisationskammergehäuse ist deshalb vorzugsweise geerdet.

Derartige Metallisierungen können durch Aluminieren oder durch Nickelplattieren einer der Seiten des Strahleinlaßfensters oder des Strahlauslaßfensters realisiert werden. Derartige aluminiumbedampfte Folien weisen eine leitende Schicht zur Vermeidung von hohen elektrischen Felddichten als Auslösepunkte von Gasentladungen auf, so daß das Auftreten von Gasentladungen minimiert wird. Darüber hinaus bilden metallisierte Folien eine glatte Oberfläche aus, die ebenfalls der Vermeidung von hohen elektrischen Felddichten aus Auslösepunkte von Gasentladungen dient.

Die großflächige plattenförmige Zählanode und die großflächige plattenförmige Hochspannungskathode bestehen vorzugsweise aus einem Gewebe, das in einem Rahmen eingespannt ist, der sich elektrisch isoliert an dem Gehäuserahmen abstützt. Dabei definieren elektrisch isolierende Abstandselemente den Abstand zwischen der Zählanode und der Hochspannungselektrode. Der Einsatz von Geweben anstelle von Folien für die Anode und die Kathode hat den Vorteil, daß mit einer größeren mechanischen Vorspannung für die Detektorebenen aus Gewebematerial gearbeitet werden kann. Damit wird die Uniformität des Signals über dem Ort und über die Ausdehnung der Detektorfläche der Ionisationskammer verbessert, was sich insbesondere bei den relativ großen Kammerquerschnitten in den Randbereichen des aktiven Volumens der Kammern vorteilhaft auswirkt.

Gegenüber großflächigen Folien als Zählanodenflächen oder - kathodenflächen haben Gewebe den Vorteil, daß sie aufgrund der höheren Vorspannmöglichkeit eine geringere Durchbiegung insbesondere bei angelegter Hochspannung aufweisen. Eine derartige Durchbiegung wird durch die gegenseitige elektrostatische Anziehung der parallel zueinander aufgespannten Folien bzw. der parallel zueinander aufgespannten Gewebeelektroden verursacht. Bei Einsatz von Geweben bleibt jedoch der Abstand der Elektroden voneinander besonders in der Mitte relativ konstant, so daß die Felddichten in vorteilhafter Weise örtlich konstant gehalten werden können.

Anstelle eines Metallfasergewebes wird vorzugsweise für die großflächige plattenförmige Zählanode und die großflächige plattenförmige Hochspannungskathode ein Gewebe aus metallisch beschichteten Kunststoffasern eingesetzt. Ein derartiges Gewebe aus Verbundfasern aus Kunststoff- und Metallbeschichtung hat den Vorteil, daß es bei geringerem Gewicht mit höheren Vorspannungen-belastet werden kann und eine niedrige Kernladungszahl für den Trägerfaden aufweist. Durch die Metallbeschichtung wird die Elektrodenfunktion realisiert und durch den Kunststoffkern der Fasern wird eine hohe mechanische Belastbarkeit erreicht, die ihrerseits Voraussetzung für eine große mechanische Vorspannung der Hochspannungsplattenelektroden ist.

Die großflächige plattenförmige Hochspannungskathode und die großflächige plattenförmige Zählanode bestehen vorzugsweise aus nickelbeschichtetem Kunststoffgewebe oder nickelbeschichtetem Polyestergewebe. Dieser Verbundwerkstoff hat nicht nur den Vorteil, daß sein Kunststoffanteil aus strahlungsbeständigen und nicht polarisierten Materialien besteht, sondern auch eine glatte Oberfläche gewährleistet, die hohe elektrische Felddichten zuläßt, ohne daß Gasentladungen ausgelöst werden.

Das Zählgas wird bei einer bevorzugten Ausführungsform im untersten Bereich in bezug auf die Schwerkraft des Ionisationskammervolumens zugeführt und im obersten Bereich abgeführt. Dazu weist weist der Gehäuserahmen der Ionisationskammer eine Zählgaseinlaßöffnung und eine Zählgasauslaßöffnung auf. Mit dieser bevorzugten Ausführungsform wird vorteilhaft erreicht, daß ein laminarer Fluß des Zählgases durch die Ionisationskammer über die Zählgaseinlaßöffnung und die Zählgasauslaßöffnung sichergestellt werden kann. Im Innern der Kammer wird das Zählgas vorzugsweise durch Edelstahlrohre mit variierbaren Aus- und Einlaßbohrungen geführt.

Ein Gasflußsensor ist vorzugsweise zur Überwachung des Zählgasdurchflusses außerhalb des Ionisationskammervolumens angeordnet, um das Kammervolumen möglichst klein zu halten. Neben der reinen Überwachung des Zählgasdurchflusses kann auch eine Zählgasregelung mit Hilfe derartiger Gasdurchflußsensoren in Zusammenwirken mit Druck- und Temperatursensoren erfolgen.

Vorzugsweise sind die zentrale Zählanode und die jeweilige Hochspannungskathode in einem Abstand von 3 bis 13 mm, insbesondere 5 mm, zueinander angeordnet und können bei Hochspannungen über 1.500 V betrieben werden. Dazu müssen die plattenförmigen Elektroden durch isolierende Teile, wie den Rahmen, die Distanzstücke, Verklebe- und Vergußmassen voneinander isoliert werden, wobei diese isolierenden Teile hohe elektrische Volumen- und Oberflächen-Widerstandswerte zwischen 10¹² bis 10¹⁴ Ω/cm³ bzw. 10¹⁶ bis 10¹⁸ Ω/cm besitzen. Damit werden vorteilhafterweise Leck- oder Kriechströme vermindert, die sonst die Messung verfälschen und die Empfindlichkeit des Gesamtsystems verschlechtern würden.

In einer weiteren bevorzugten Ausführungsform wird die Ionisationskammer zu einem Ionisationskammersystem für Ionenstrahlen ausgebaut. Dazu werden mehrere Ionisationskammern des erfindungsgemäßen Typs in Strahlrichtung hintereinander angeordnet und zu einem System zur Intensitätsüberwachung eines Schwerionentherapiestrahls eingesetzt. Aufgrund der hohen Sicherheitsstandards, die bei Therapiestrahlen einzuhalten sind, werden mindestens zwei Ionisationskammern in Ionisationsstrahlrichtung in Reihe hintereinander zur Überwachung der Einzeldosis eines Volumenelementes und der Schichtdosis einer gescannten Schicht angeordnet und zur unabhängigen Überwachung einer Gesamtdosis eines Behandlungszyklus unabhängig von der Ionisationskammer zur Überwachung der Einzeldosis und der Schichtdosis eingesetzt.

Aufgrund der flachen Bauweise einer Einzelionisationskammer hat dieses Ionisationskammersystem den Vorteil, daß es in Strahlrichtung den kleinsten Raumbedarf aufweist, während es sich quer zum Strahl über die volle Scanfläche erstreckt. Da mit einem Rasterscanner der Ionenstrahl das Zielvolumen volumenelementweise und schichtweise abtastet, wird vorteilhaft mit einer ersten Ionisationskammer in dem Ionisationskammersystem die Einzeldosis pro Volumenelement überwacht und durch Summieren aller Einzeldosen einer gescannten Schicht die Schichtdosis überwacht. Eine von der ersten Ionisationskammer unabhängige zweite Ionisationskammer kann vorteilhaft die Gesamtdosis eines Behandlungszyklus überwachen. Anstelle der bevorzugten zwei Ionisationskammern können auch drei Ionisationskammern hintereinander geschaltet werden, die dann die Einzeldosis eines Volumenelements, die Schichtdosis einer gescannten Schicht und die Gesamtdosis eines Behandlungszykluses jeweils überwachen.

In einer weiteren bevorzugten Ausführungsform überwacht die erste und zweite Ionisationskammer die Pixeldosis und die Schichtdosis redundant. Die zweite Ionisationskammer überwacht somit die erste Ionisationskammer. Eine dritte Ionisationskammer ist an eine andere Elektronik angeschlossen und überwacht integrale Werte, wie das Unterschreiten einer maximalen Dosis im Behandlungsplan.

Zur Erhöhung der Sicherheit kann die Einzeldosis für ein Volumenelement in der ersten und der zweiten Ionisationskammer eines Ionisationkammersystems, das drei Ionisationskammern umfaßt, gemessen und die Ergebnisse der ersten und der zweiten Ionisationskammer verglichen werden, so daß bei einer Abweichung der Meßdaten von der ersten und der zweiten Ionisationskammer über einen vorgegebenen Toleranzbereich hinaus eine Schnellabschaltung des Ionentherapiesystems ausgelöst werden kann. Ein derartiger Vergleich erhöht die Betriebssicherheit der ersten und der zweiten Ionisationskammer. In gleicher Weise kann die Schichtdosis einer zu bestrahlenden Schicht (auch Bestrahlungsschicht genannt) mit der zweiten und dritten Ionisationskammer des Ionisationskammersystems mit drei Ionisationskammern gemessen und verglichen werden, so daß beim Überschreiten eines vorgegebenen Toleranzbereichs der Messergebnisse der zweiten und dritten Ionisationskammer eine Sicherheitsabschaltung des Ionenstrahltherapiesystems ausgelöst werden kann.

Eine Vorrichtung zur Intensitätsüberwachung eines Schwerionenstrahls mit erfindungsgemäßen Ionisationskammern oder mit dem erfindungsgemäßen Ionisationskammersystem weist folgende Merkmale auf:
a) Mittel zum Messen der Intensitätsdosis eines Bestrahlungsvolumenelements eines geplanten Bestrahlungsrasters für eine Bestrahlungsschicht mittels einer ersten Ionisationskammer,
b) Mittel zum Überwachen des gemessenen Wertes der Intensitätsdosis eines Bestrahlungsvolumenelementes durch eine zur ersten Ionisationskammer in Reihe hintereinander angeordnete zweite Ionisationskammer,
c) Mittel zum Vergleichen des Meßwertes der ersten Ionisationskammer mit dem Überwachungswert der zweiten Ionisationskammer und Freigabe zur Bestrahlung des nächsten Bestrahlungsvolumenelementes eines geplanten Bestrahlungsrasters einer Bestrahlungsschicht bei Übereinstimmung mit einer vorgegebenen Sollwertbreite der Bestrahlungsintensität beider Werte,
d) Mittel zum Notabschaltung der Bestrahlungsbehandlung bei Überschreiten der vorgegebenen Sollwertbreite und Nachregeln der Intensität bei Unterschreiten der vorgegebenen Sollwertbreite,
e) Mittel zum Wiederholen der Schritte für die nächstfolgenden geplanten Behandlungsschichten, bis das zu bestrahlende Gewebevolumen abgerastert ist,
f) Mittel zum Integrieren aller gemessenen Bestrahlungsdosen der Überwachungswerte in einer dritten Ionisationskammer, die in Reihe hintereinander zu der ersten und zweiten Ionisationskammer angeordnet ist zur Überwachung der Gesamtstrahlungsbelastung eines Gewebevolumens während eines Behandlungszyklus.

Mit dieser Vorrichtung wird vorteilhaft die Anzahl der Teilchen oder Schwerionen, die pro Sekunde von einem Beschleuniger extrahiert werden und als Ionenstrahl zur Tumortherapie eingesetzt werden, gemessen. Die Anzahl der Teilchen unterliegt starken zeitlichen Schwankungen und muß deshalb in Echtzeit unmittelbar mit diesen Ionisationskammern während der Bestrahlung gemessen werden. Der Strom, der am Ausgang der Kammern gemessen wird, ist bei gleichbleibender Teilchenenergie proportional zum Ionenstrahlstrom. Bei typischen Strahlströmen des Beschleunigers liegen die Ströme, die aus den Ionisationskammern kommen, im Bereich von µA.

Die Ansprechgeschwindigkeit der Ionisationskammern wird durch die Driftzeit der ionisierten Zählgasmoleküle in der Ionisationskammer begrenzt und hat eine Verzögerungskonstante in der Größenordnung von etwa 10 µs. Eine Meßelektronik wandelt den Strom aus der Ionisationskammer in eine proportionale Frequenz von Impulsen um. Dazu werden aus dem Stromsignal Spannungssignale erzeugt und durch eine Amplitudenfrequenzwandlung werden aus dem Spannungssignal Impulse gebildet, deren Frequenz proportional zur Spannung ist. Demnach entspricht ein Impuls einer bestimmten Ladung, die in der Ionisationskammer erzeugt wurde, diese wiederum wurde von einer bestimmten Teilchenzahl des Ionenstrahls erzeugt. Die Anzahl der erzeugten Impulse ist somit proportional zur Anzahl der Ionen, die durch die Ionisationskammer fließen.

Somit kann mit der Vorrichtung vorteilhaft die Intensitätsdosis eines Bestrahlungsvolumenelementes, die Intensitätsdosis einer gesamten Bestrahlungsschicht und schließlich die Intensitätsdosis eines ganzen Behandlungszykluses überwacht werden. Die Vorrichtung beinhaltet auch eine sicherheitsrelevante Redundanz, indem praktisch in zwei Ionisationskammern sowohl die Intensitätsdosis eines Bestrahlungsvolumenelementes als auch die Intensitätsdosis einer Bestrahlungsschicht gemessen wird und die Meßwerte miteinander unmittelbar verglichen werden, so daß bei nicht tolerierbaren Abweichungen eine Notausschaltung der Anlage veranlaßt werden kann. Auch ist es möglich, an jede der drei Ionisationskammern eine Summationselektronik anzubauen, so daß alle drei Ionisationskammern gleichzeitig und parallel die Gesamtdosis eines Bestrahlungszykluses eines Bestrahlungsvolumens überwachen können. Somit wird durch die erfindungsgemäßen Ionisationskammern und insbesondere durch das Ionisationskammersystem von drei in Ionenstrahlrichtung hintereinander angeordneter Ionenstrahlkammern eine größtmögliche Sicherheit und Zuverlässigkeit beim Bestrahlen eines Tumorvolumens mit Ionenstrahlen erreicht.

Bei einer bevorzugten Weiterbildung wird die Überwachungsfunktion der ersten und der zweiten Ionisationskammer von einer einzigen Ionisationskammer wahrgenommen. Damit vermindert sich zwar die Redundanz der Intensitätsüberwachung, aber der Raumbedarf für die Detektorsysteme in Form von Ionisationskammern wird vorteilhaft vermindert.

Weitere Vorteile, Merkmale und Anwendungsmöglichkeiten der Erfindung werden nun anhand von Ausführungsbeispielen unter Bezugnahme auf die beigefügten Zeichnungen näher erläutert.

Fig. 1 zeigt einen prinzipiellen Aufbau in perspektivischer Ansicht einer bevorzugten Ausführungsform der erfindungsgemäßen Ionisationskammer.

Fig. 2 zeigt einen schematischen Querschnitt der bevorzugten Ausführungsform nach Fig. 1.

Fig. 3 zeigt die Homogenität des Ortsansprechsverhaltens einer Ausführungsform der erfindungsgemäßen Ionisationskammer in Y-Richtung.

Fig. 4 zeigt die Homogenität des Ansprechsverhaltens einer Ausführungsform der erfindungsgemäßen Ionisationskammer in X-Richtung.

Fig. 5 zeigt den prinzipiellen Aufbau eines Ionisationskammersystems im Querschnitt.

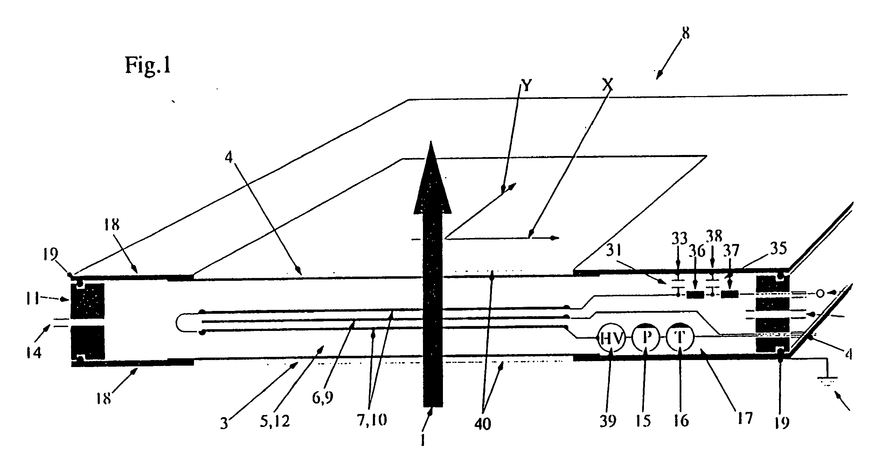

Fig. 1 zeigt einen prinzipiellen Aufbau in perspektivischer Ansicht einer bevorzugten Ausführungsform der erfindungsgemäßen Ionisationskammer 8. Die hier gezeigte Parallel-Platten-Ionisationskammer 8 dient der Überwachung und Steuerung der Patientenbestrahlung im Rahmen der Tumortherapie mit schweren Ionen. Sie besteht im wesentlichen aus einem Kammergehäuse 2, einem Strahleneinlaßfenster 3 und einem Strahlenauslaßfenster 4, einem mit Zählgas gefüllten Kammervolumen 5, einer Hochspannungsanode 6 und einer Hochspannungskathode 7, die beidseitig der Hochspannungsanode 6 angeordnet ist. Die Ionisationskammer ist für den medizinischen Bereich gedacht und sandwichartig flach aus plattenförmigen großflächigen Strukturen der einzelnen Komponenten, die orthogonal zum Ionenstrahl 1 ausgerichtet sind, aufgebaut. Zu den Komponenten gehört eine zentral angeordnete großflächige orthogonal ausgerichtete plattenförmige Zählanode 9, die von einer großflächigen plattenförmigen Hochspannungskathode 7 aus zwei parallelen Kathodenplatten 10 beidseitig umgeben ist. Das Kammergehäuse 2 besteht im wesentlichen aus einem Gehäuserahmen 11, der ein quadratisches Ionisationskammervolumen 12 umrahmt und auf dem das Strahleinlaßfenster 3 und das Strahlauslaßfenster 4 gasdicht angebracht sind. Eine derartige Ionisationskammer 8 muß im medizinischen Bereich eine hohe Sicherheit, eine hohe Zuverlässigkeit und große Wartungsfreundlichkeit aufweisen. Deshalb erfüllt die vorliegende Ausführungsform die folgenden Rahmenbedingungen und Anforderungen, wobei für diese Ausführungsform die folgenden Parameter der Bestrahlung maßgebend sind:

| | | |
|---|---|---|
| Strahl-Sorte: | Protonen, Kohlenstoff Sauerstoff | ¹²C⁶⁺ (vorzugsweise) |
| - Energie: | 80 ... 430 MeV/u | in 253 Schritten |
| - Focus: | 4 ... 10 mm | in 4 bis 7 Schritten |
| - Intensität: | 2˙10⁶ - 4˙10¹⁰ Teilchen/ Extraktion | in 10 bis 20 SChritten |
| | | |
| Bestrahlungs-Geometrie: | 200 x 200 mm² | in der Isozentrums-Ebene |
| | | |
| Charakteristische Zeiten: | 1 - 10 s | Ionenstrahl-Extraktionszeit |
| | 2 - 4 s | Strahlpause |
| | ≥ 1000 µs | pro Bestrahlungspunkt eines Volumenelements |
| | < 1000 µs | für den Strahlabbruch |
| | 100 µs - 250 µs | pro Positionsmessung |
| | 10 µs - 15 µs | pro Intensitätsmessung |

Während einer Extraktionsphase von etwa 2 s durchfliegen die aus einem Ionenbeschleunigersystem, wie einem Schwerionensynchrotron, extrahierten Kohlenstoffionen die Ionisationskammer und geben bei Stößen mit dem Zählgas einen Teil ihrer kinetischen Energie an dieses ab. Ein Teil dieser Energie führt zur Erzeugung von Elektron-Ion-Paaren. Die Anzahl der Elektron-Ion-Paaren ist proportional zur Menge der hindurchgeflogenen Ionen des Ionenstroms. Die somit im aktiven Ionisationskammervolumen 12 erzeugten Ladungen werden in einem angelegten elektrischen Feld getrennt und von der nachfolgenden Meßelektronik registriert und ausgewertet. Dazu besteht der aktive Bereich nur aus den Räumen zwischen den Elektroden. Nur die dort erzeugten Ladungen werden erfaßt und gemessen. Die in den Bereichen zwischen Elektroden und Kammerfenstern erzeugten Ladungen werden auf den Hochspannungsebenen 7 bzw. den Fenstern 3, 4 abgesaugt und tragen somit nicht zur Signalbildung bei. Sie werden von der Messung ausgeschlossen, da die in diesen Bereichen durch die Geometrie bedingten Feldinhomogenitäten keine repräsentativen Intensitätsmessungen zulassen.

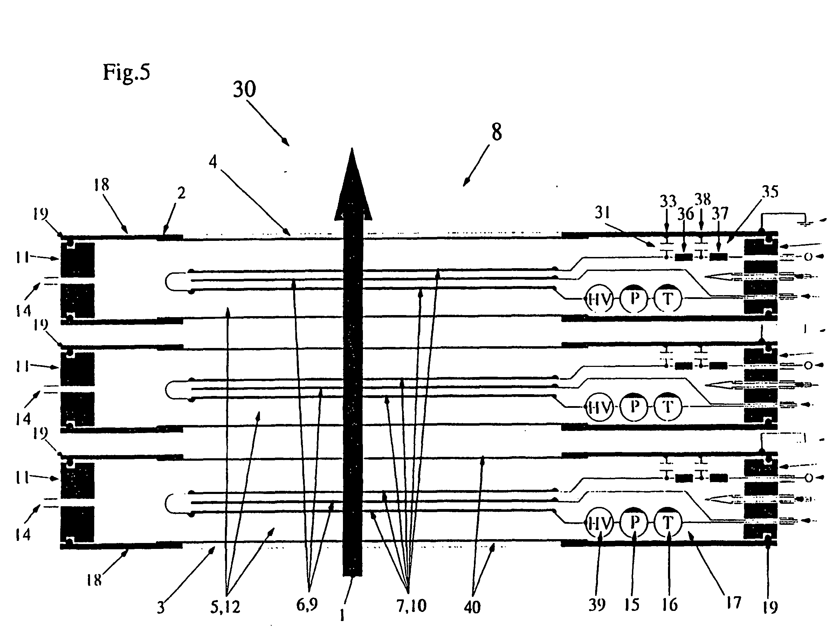

Bei einem Ionisationskammersystem 30 von drei Ionisationskammern 22, 23 und 24 geschieht die Auswertung in zwei dieser Ionisationskammern 22 und 23 alle 12 µs. Über einen Vergleich dieser Werte mit den zuvor im Rahmen einer Bestrahlungsplanung ermittelten Sollwerten dient diese Information zur Regelung der Scangeschwindigkeit eines Rasterscanners am Behandlungsplatz. Die dritte in Fig. 5 gezeigte Ionisationskammer 24 ist von baugleichem Typ und dient der Bestimmung der Gesamtteilchenzahl für einen Isoenergieschritt, d.h. für eine Bestrahlungsschicht, sowie für die gesamte Strahlendosis eines Bestrahlungszyklus für ein Tumorvolumen. Durch die Überwachung dieser integralen Werte, nämlich der Bestrahlungsschicht und des Bestrahlungsvolumens ist eine weitere Sicherheit für das Abtasten des Tumorvolumens im Patienten gegeben.

Durch das Abtastgerät, einem Scanner, wird am Ort der Ionisationskammern 22, 23, 24 ein Bereich von ca. 190 x 190 mm² abgedeckt. Demzufolge weisen die Ionisationskammern 22, 23, 24 dieser bevorzugten Ausführungsform einen großen aktiven Querschnitt mit einer Öffnungsweite für das Strahleinlaßfenster 3 und das Strahlauslaßfenster 4 von 210 x 210 mm² auf. Durch den Einsatz von Materialien mit relativ hohem Elastizitätsmodul lassen sich diese Abmessungen insbesondere im Zähleraufbau vorteilhaft bewältigen.

Die Ionisationskammer 8, 22, 23, 24 ist jeweils als Faraday-Käfig ausgebildet, womit vorteilhaft elektromagnetische Einstreuungen vermieden werden. Bei der Verkabelung der plattenförmigen großflächigen Hochspannungselektroden 6 und 7 werden Erdschleifen außerhalb der Ionisationskammern konstruktiv vermieden, um elektromagnetische Einstreuungen zu minimieren.

Der Gehäuserahmen 11 der Ionisationskammer 8 besteht in dieser Ausführungsform aus einem Aluminiumvollmaterial, die Rahmen der Fenster 18 bestehen aus Edelstahl und die Fensterfolien des Strahleinlaßfensters 3 und des Strahlauslaßfensters 4 bestehen aus metallisierten Kunststoffolien von etwa 25 µm Dicke, die elektrisch leitend in den Rahmen 18 der Fenster eingeklebt sind. Alle leitenden, nicht elektrische Spannungen führenden Teile sind zentral über den Erdanschluß 25 geerdet. Soweit als möglich werden für Materialien, durch die der Ionenstrahl 1 in dem aktiven Ionisationskammervolumen 12 hindurchstrahlen muß, Materialien mit niedrigen Kernladungszahlen eingesetzt, vorzugsweise Kunststoffe aus Wasserstoff und Kohlenstoff und mit möglichst geringer Dicke und damit Massenbelegung im eigentlichen Ionenstrahlengang. Das hat den Vorteil, daß eine Verschlechterung der Strahlqualität, wie etwa durch Projektilfragmentation und Winkelstreuung der Strahlteilchen, durch diese Materialien minimiert wird. Darüber hinaus wurden für derartige Materialien nur strahlungsbeständige und nicht polarisierbare Stoffe in dieser Ausführungsform eingesetzt.

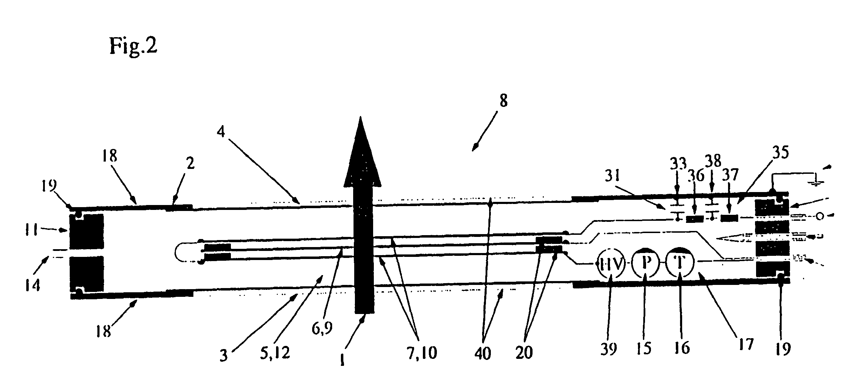

Die isolierenden Teile, die in Fig. 2 gezeigt werden, wie der Rahmen 20 der Zählanode 6 und der Hochspannungskathode 7 sowie die Abstands-elemente zwischen diesen Rahmen und die Klebe- und Vergußmassen weisen einen Volumen- und Oberflächenwiderstandswert von 10¹² bis 10¹⁴ Ω/cm³ bzw. 10¹⁶ bis 10¹⁸ Ω/cm auf. Dieser hohe Oberflächen- und Volumenwiderstand hat den Vorteil der Verringerung von Dunkelströmen, die die Messungen verfälschen und die Empfindlichkeit des Gesamtsystems verschlechtern würden.

Leitende Teile sind in dieser Ausführungsform mit glatten Oberflächen ausgestattet, womit vorteilhaft hohe elektrische Felddichten als Auslösepunkte von Gasentladungen vermieden werden. Die Folien für das Strahleinlaßfenster 3 und das Strahlauslaßfenster 4 sind deshalb mit Aluminium bedampft und die Hochspannungsanode 6 bzw. Zählanode 9 und die Hochspannungskathode 7 bzw. die Hochspannungskathodenplatten 10 sind deshalb aus Gewebe mit hoher Maschenzahl gefertigt und mit Nickel beschichtet, um auch hier relativ glatte Oberflächen zu realisieren.

Fig. 2 zeigt einen schematischen Querschnitt der bevorzugten Ausführungsform nach Fig. 1. Entsprechend der ebenen Bestrahlungsgeometrie ist die Ionisationskammer 8, wie es die perspektivische Ansicht der Fig. 1 zeigt, als Parallelplatten-Ionisationskammern8, 22, 23, 24 ausgebildet. Die Ionisationskammer 8, wie in Fig. 2 gezeigt, hat einen symmetrischen Aufbau ihrer Zählanode 9 zwischen zwei Hochspannungskathodenplatten 10 mit einem Abstand von jeweils 5 mm in Strahlrichtung. Dieses gewährleistet vorteilhaft eine exakte Definition des aktiven Volumens. Ferner ist vorteilhaft bei der gleichen Driftzeit der Elektron-Ion-Paare das Signal und damit auch das Signal/Rausch-Verhältnis vorteilhafterweise doppelt so groß wie bei nichtsymmetrischem Aufbau aus beispielsweise zwei Folien. Die nichtaktive und damit tote Zone der Ionisationskammer beträgt 8 - 22 mm in Strahlrichtung, so daß in Strahlrichtung eine Gesamtausdehnung der Ionisationskammer 8 in dieser Ausführungsform 18 - 32 mm beträgt.

Die Zählanoden-9, Abstandselemente-21, und Hochspannungskathodeneberren 10 sind aufeinander ausgerichtet, um eine genaue Definition des aktiven Volumens 12 zu erreichen. Dazu werden diese Ebenen planparallel und senkrecht zum Ionenstrahl 1 mittels engtolerierter, gemeinsamer Aufhängepunkte der Ebenen in Strahlrichtung durch entsprechende Distanzstücke angeordnet.

Unvermeidliche Leckströme zwischen hochspannungsführenden Teilen, wie der Kathodenplatten 10 und der Zählanode 9, werden durch geerdete Schutzelektroden von den Signalelektroden ferngehalten und abgeleitet.

Für die Realisierung der Kammerebenen wie den Kathodenplatten 10 und der Zählanode 9 wurde in dieser Ausführungsform ein nikkelbeschichtetes Polyestergewebe eingesetzt. Damit wird eine hohe Beständigkeit der Kammerebenen, insbesondere der Kammerelektroden, gegen große Ionisationsdichten infolge hoher Teilchendichten und hoher Teilchenraten im Ionentrahl 1 während der Extraktion aus einem Sychrotron erreicht.

Das nickelbeschichtete Polyestergewebe der Elektroden besteht vorzugsweise aus einem Siebdruckgewebe mit einer Faserstärke von 38 µm, einem Faserabstand von 54 µm und einer offenen Fläche von 34,45 %. Dabei setzt sich die Faserstärke aus einem 36 µm starken Polyesterkern mit einer 1 µm dicken Nickelbeschichtung zusammen. Die mittlere Dicke des nickelbeschichteten Polyestergewebes beträgt 62 µm und die maximal äquivalente Reichweite der Kohlenstoff-Strahlteilchen in diesem Gewebe beträgt ca. 100 µm.

Sowohl der Einsatz von Gewebe an sich als auch die Wahl von Polyester als Träger und Nickel als Besphichtungsmaterial bieten Vorteile, die im folgenden beschrieben werden.

Einerseits ergeben sich bei Verwendung von Gewebeebenen gegenüber Folien folgende Vorteile:
a) Die Gestaltung der an der Signalbildung beteiligten Detektorebenen aus Gewebematerial läßt eine größere mechanische Vorspannung von über 10 Ncm gegenüber Folienebenen zu.
b) Die Uniformität des Signals über dem Ort wird damit verbessert, was sich insbesondere bei den relativ großen Kammerquerschnitten in den Randbereichen des aktiven Volumens der Ionisationskammer 8 positiv auswirkt.

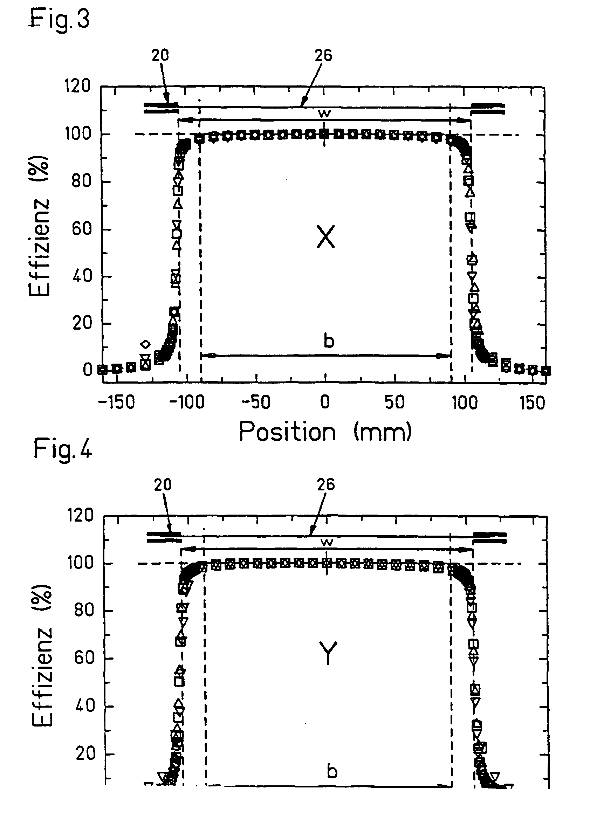

Fig. 3 zeigt dazu die Homogenität des Ortsansprechsverhaltens einer Ausführungsform der erfindungsgemäßen Ionisationskammer 8 in Y-Richtung, und Fig. 4 zeigt dazu die Homogenität des Ortsansprechverhaltens einer Ausführungsform der erfindungsgemäßen Ionisationskammer 8 in X-Richtung.

In den Fig. 3 und 4 ist auf der Abszisse der jeweilige Abstand in mm vom Zentrum der Ionisationskammer 8 aufgetragen und auf der Ordinate ist die erreichte relative Signalhöhe in % gezeigt. Die punktierte Linie im oberen Bereich der Figuren 3 und 4 symbolisiert das nickelbeschichtete Gewebe der Elektroden 26 und 27, das zwischen dem Rahmen 20, der eine lichte Weite w von 210 mm aufweist, aufgespannt ist. Deutlich erkennbar ist, daß über eine aktive Volumenbreite b von 190 mm das Ortsansprechsverhalten der Kammer, d.h. das Signal, äußerst homogen ist, und erst zum Rand, d.h. zu den Einspannrahmen 20 der Gewebe 26 und 27 hin steil abfällt. Diese Uniformität und Homogenität des Signals macht sich insbesondere bei großen Beträgen der angelegten Hochspannung bemerkbar, bei denen Ionisationskammern mit Folien bereits zu einer merklichen Durchbiegung der Ebenen infolge gegenseitiger elektrostatischer Anziehung neigen. Durch die höhere mechanische Vorspannung der Gewebe in ihren Rahmen 20 ist die Durchbiegung derartiger Elektroden geringer als bei Elektroden aus Folien. Insbesondere deshalb, weil sich der Abstand der Elektroden voneinander in der Mitte gegenüber Folienelektroden kaum verringert und damit bei Gewebeelektroden die Felddichten örtlich konstant bleiben.

Die Auslösung von Funken speziell am Punkt größter Durchbiegung in der Mitte der Ionisationskammer 8 wird zu größeren Hochspannungen hin verschoben, bei denen im Fall von Folienkammern innerhalb kürzester Zeit eine Zerstörung der Metallisierung auftreten würde.

Dieses hat vergleichsweise den Vorteil von höheren maximalen Betriebsspannungen der Gewebekammern von ca. 1.900 V gegenüber Gewebekammern mit gleichen Elektrodenabstand aus Folienelektroden von ca. 1.000 V. Derart erhöhte Betriebsspannungen wirken sich günstig auf das Ladungssammelverhalten der Ionisationskammer 8 aus, da sich die Driftseiten verkürzen, womit vorteilhaft eine verkürzte Antworts-/Reaktionszeit des Gesamtsystems möglich wird. Dieser Vorteil ist insbesondere aus Sicherheitsgründen wichtig, für die ein schneller Extraktionsabbruch im Fehlerfall gefordert wird.

Des weiteren wird durch die höhere mechanische Spannung und die größere Masse der Gewebeelektroden in dieser bevorzugten Ausführungsform die Eigenfrequenz der Ionisationskammer 8 zu höheren Frequenzen hin verschoben. Sie liegt bei Ionisationskammern mit Gewebeelektroden dieser Ausführungsform bei ca. 500 Hz. Diese hohe Frequenz ist wesentlich günstiger als bei Ionisationskammern mit Folienelektroden, bei denen bereits die kleinsten Erschütterungen durch lautes Reden oder unvermeidliche Körperschallschwingungen durch die Vakuumpumpen zu einer teilweise erheblichen Verfälschung des Meßsignals führen können.

Somit wird durch die Gewebeelektroden dieser bevorzugten Ausführungsform der Erfindung der sonst störende Mikrophonie-Effekt-bei Ionisationskammern 8, 22, 23, 24 vermieden.

Ein weiterer Vorteil der Gewebeelektroden liegt in der Gasdurchlässigkeit dieser Konstruktion. Dadurch wird der laminare Fluß des Zählgases durch und zwischen den Hochspannungs- und Signalebenen erheblich verbessert, was ebenfalls der Langzeitstabilität dient. Ober diese Vorteile hinaus bietet der Einsatz von metallisierten Polyestergeweben gegenüber Vollmetallgeweben den Vorteil einer geringeren Strahlbeeinflussung, da wegen der kleineren Kernladungszahl des Verbundmaterials und der im Mittel geringeren Massenbelegung weniger Sekundärprodukte oder Fragmente beim Durchgang des Ionenstrahls durch das Gewebe entstehen und auch die Winkelstreuung geringer ist, was die Belastung von gesundem Gewebe bei einer Tumorbestrahlung oder Tumortherapie vermindert.

Aufgrund des hohen Schmelzpunktes und der ausgezeichneten Haftung einer Nickel-Metallisierung hat das nickelbeschichtete Gewebe darüber hinaus den Vorteil größerer Betriebssicherheit, da die Schädigung durch gelegentliche. Funken geringer ausfällt als bei einer Aluminiumbeschichtung. Somit verringert auch die gute chemische Beständigkeit der Nickelbeschichtung die Alterungseffekte infolge gecrackter Zählgase, wie sie bei der Auslösung von Gasentladungen auftreten. Somit trägt die chemische Beständigkeit des Nickels oder der Nickelbeschichtung zur Erhöhung der Gebrauchsdauer oder Lebensdauer der eingesetzten Gewebeelektroden und damit zur Erhöhung der Lebensdauer der Ionisationskammer bei.

Das in dieser Ausführungsform eingesetzte Siebdruckgewebe ist gegenüber Metallgeweben, beispielsweise aus Titan, äußerst kostengünstig. Ferner weisen Siebdruckgewebe eine gleichbleibende Qualität auf, was ebenfalls einen großen Vorteil für die Fertigung von Ionisationskammern 8, 22, 23, 24 darstellt.

Fig. 5 zeigt den prinzipiellen Aufbau eines Ionisationskammersystems 30 im Querschnitt. Dieses Ionisationskammersystem 30 ist aus drei in Richtung eines Ionenstrahls 1 hintereinanderliegenden Ionisationskammern 22, 23 und 24, die den in Fig. 1 und 2 abgebildeten Ionisationskammern 8 entsprechen, zusammengesetzt. Mit diesen drei voneinander unabhängigen Ionisationskammern 22, 23 und 24 wird die sicherheitstechnisch erforderliche Redundanz der Strahlstrommessung erreicht.

Der verfügbare Platz am Strahlaustritt in einem Bestrahlungsraum vor einem Patienten ist eng begrenzt. Deshalb sind diese Ionisationskammern 22, 23, 24 in Strahlrichtung schmal (mit je 35 mm Tiefe) und in einem kompakten Aufbau installiert. Das Ionisationskammersystem 30 ist auf einer seitlich angebrachten, nicht gezeigten gemeinsamen Grundplatte montiert, so daß die Grundplatte nicht die Detektorfläche bzw. das Strahleinlaßfenster 3 oder das Strahlauslaßfenster 4 abdeckt.

Auf der gemeinsamen Grundplatte sind die Ionisationskammern8, 22, 23, 24 getrennt austauschbar. Zur Ausrichtung auf die Strahlmitte werden die Mittenanrisse an den jeweiligen Gehäuserahmen 11 und auf den Fenstern der Ionisationskammern 22, 23 und 24 mit dem im medizinischen Bestrahlungsraum durch ein Lasersystem abgebildeten Koordinatensystem zur Deckung gebracht. Dabei wird das Ionisationskammersystem 30 als Ganzes mittels isolierender Justageschrauben an der Grundplatte positioniert. Die dabei erzielbare Genauigkeit ist ± 1 mm.

An jeder Isolationskammer 8, 22, 23, 24 sind Paßvorrichtungen angeordnet, die eine Reproduktion der Justage nach einem eventuellen Ausbau einzelner Ionisationskammern 22, 23, 24 gewährleisten. Jede der Ionisationskammern 22, 23 und 24 weist an zwei Stellen leitend eingeklebte Messinggewindestücke auf mit einem Übergangswiderstand von kleiner als 3 Ω. Mittels dieser Messinggewindestücke wird eine gute Erdung des Gesamtsystems gewährleistet.

Die Hochspannungsversorgung der symmetrisch angeordneten Hochspannungskathoden 7 wird über entsprechende Hochspannungsdurchführungen 32 im Gehäuserahmen 11, den Hochspannungskathoden 7, die als Kathodenplatten 10 symmetrisch zur Zählanode 9 des aktiven Ionisationskammervolumens 12 angeordnet sind, zugeführt. Die Überwachung der Hochspannung durch das im folgenden beschriebene Sensorelement 39 beinhaltet auch die Feststellung eines Kabelbruches innerhalb der Kammer durch Messung des Durchgangswiderstandes zwischen den Anschlüssen 11 und dem (nicht dargestellten) Spannungsteiler an der Ebene 10. Die Hochspannung wird durch ein Filterglied 31 mit Pufferkondensator 33, 34, wie in den Fig. 1, 2 und 5 gezeigt, stabilisiert, wobei das Filterglied 31 in der Ionisationskammer 8, 22, 23, 24 angeordnet ist. Mit diesem Filterglied 31 in der Ionisationskammer 8, 22, 23, 24 werden vorteilhaft Signalschwankungen und Meßwertverfälschungen infolge schwankender Betriebsspannungen bei hohen Meßströmen vermieden.

Der Ausgangswiderstand der Hochspannungsversorgung beträgt typischerweise 10 MΩ. Die Zeitkonstante der Kombination Hochspannungsgerät plus Kabel plus Ionisationskammer liegt in der Größenordnung von 1 ms, was wesentlich länger ist als die typische Zeitkonstante der Extraktionsunterstruktur aus beispielsweise einem Synchrotron von einigen 100 µs. Als gesammelte Ladung treten in 1 ms ca. 10⁻⁸ As auf. Bei einem maximalen Spannungsabfall unter 1 V am Pufferkondensator 33, 34 ist die Kapazität von 10 nF ausreichend. In den bevorzugten Ausführungsformen der Figuren 1, 2 und 5 sind Hochspannungskondensatoren mit 50 nF als Pufferkondensator 33, 34 angeordnet. Zusätzlich weist die Ionisationskammer 8 22, 23, 24 der Ausführungsform nach Fig. 1, 2 und 5 im Kammervolumen 5 ein T-Filter 35 auf, das aus zwei Widerständen 36, 37 von je 1 MΩ und einem Kondensator 38 von beispielsweise 1,2 nF besteht, auf. Dieses T-Filter 35 hat den Vorteil, daß es Brummeinstreuungen durch Sieben von den Hochspannungselektroden fernhält.

Zur Messung und Überwachung der Hochspannung wird die Hochspannung über einen hochspannungsfesten Spannungsteiler mit festem Teilerverhältnis (beispielsweise 1 : 1000) in der Ausführungsform nach Fig. 1, 2 und 5 realisiert. Die Messung der Betriebsspannung erfolgt direkt an der Hochspannungskathode 7 mittels einem Hochspannungssensor 39. Die Überwachung der Hochspannung mittels des Sensors 39 wird vorzugsweise zur Überwachung der Ladungssammeleffizienz der Ionisationskammer 8, 22, 23, 24 eingesetzt, da von der Höhe der Betriebsspannung die registrierte Strahlteilchenzahl abhängt.

In den Ausführungsformen nach Fig. 1, 2 und 5 weist die Ionisationskammer 8, 22, 23, 24 eine spezielle Gasversorgung mit Zählgas gleichbleibender Qualität und Zusammensetzung auf. Gegenüber einer Luftfüllung, die aufgrund der Luftfeuchte die Sensitivität einer Ionisationskammer beeinflußt und die Reproduzierbarkeit der Messungen herabsetzt, wird hier als Zählgas ein trockenes Gasgemisch eingesetzt. Als Zählgas oder Kammergas wird in dieser Ausführungsform nach Fig. 1, 2 und 5 ein Argon oder Krypton/CO₂-Gemisch 80/20 % verwendet. Dieses Gasgemisch hat den Vorteil, daß es einen ausreichenden W-Wert als Zählgas aufweist. Der W-Wert dieses Gasgemisches liegt ausreichend niedrig zur Erreichung ausreichender Signale bei kleinen Teilchenraten (beispielsweise minimal 10⁶ T/s) und für ein gutes Signal-/Rauschverhältnis sowie ausreichend hoch zur Abdeckung eines größeren dynamischen Bereichs in den Teilchenraten (z.B. Faktor 100, d.h. maximal 10⁸ T/s).

Das Kammervolumen 5 ist gegenüber der Umgebung hermetisch abgeschlossen, so daß das Zählgas wegen der Empfindlichkeit des Signals gegenüber Verunreinigung rein gehalten werden kann. Auch bei Nichtbetrieb kann deshalb ein Gasaustausch infolge von Diffusion mit der Umgebung nicht oder nur minimal stattfinden. Die hermetische Abdichtung des Kammervolumens 5 wird mittels O-Ring-Dichtungen 19 der Strahleinlaßfenster 3 und Strahlauslaßfenster 4 gegenüber dem Gehäuserahmen 11 erreicht.

Darüber hinaus sind in dem Gehäuserahmen 11 selbstschließende, verwechslungssichere Gasdurchführungen in den Zählgaseinlaßöffnungen 13 und Zählgasauslaßöffnungen 14 vorgesehen sowie gasdichte elektrische Durchführungen 32 sowie Durchführungen 45 für die Sensorik in den Gehäuserahmen 11 eingebaut. Die Fensterfolien 40 des Strahleinlaßfensters 3 und des Strahlauslaßfensters 4 sind in die Rahmen 18 der Fenster gasdicht eingeklebt. Für die gesamten Bauelemente, die im Kammervolumen 5 eingesetzt werden, werden nichtgasende Materialien verwendet.

Die Zählgaseinlaßöffnung 13 ist in bezug auf die Schwerkraft unten angeordnet und die Zählgasauslaßöffnung 14 entsprechend oben angeordnet. Darüber hinaus weisen die Ionisationskammern 8, 22, 23, 24 Röhren mit Bohrungen verschiedenen Durchmessers auf, so daß im Zusammenwirken dieser Bohrungen in der Anordnung der Zählgaseinlaßöffnung 13 und der Zählgasauslaßöffung 14 ein laminarer Fluß zwischen den Detektorebenen gewährleistet werden kann. Ein ausreichender laminarer Fluß des Zählgases von mehr als zwei 1/h durch die Ionisationskammern hat den Vorteil, reproduzierbare und langfristig stabile Meßwerte zu erzielen. Da die Dichte des verwendeten Gasgemisches höher ist als die der normalen Luft, ist ferner die spezielle Anordnung der Zählgaseinlaßöffnung 13 und der Zählgasauslaßöffnung 14 zur Erzielung eines laminaren Flusses von Bedeutung, da sonst nichtentfernbare Luftpolster einen laminaren Fluß behindern und die Reinheit des Zählgases herabsetzen würden.

Um ein Verunreinigen des Zählgases durch Eindringen von Fremdgasen zu erschweren, werden die Ionisationskammern 8, 22, 23, 24 mit leichtem Überdruck gegenüber der umgebenden Luft betrieben. Die Größe des Gasrückflusses aus den Ionisationskammern 8, 22, 23, 24 wird durch einen Sensor im Auslaß überwacht, so daß Undichtigkeiten feststellbar sind.

In der Ionisationskammer 8, 22, 23, 24 sind in dem Kammervolumen 5 Sensoren für Gasdruck 15 und für die Gastemperatur 16 eingebaut. Aus diesen Meßwerten kann unmittelbar die Gasdichte bestimmt werden, die wiederum in den Wert der Strahlteilchenzahl eingeht. Durch den Sensor für den Gasdruck 15 und den Sensor für die Temperatur 16 kann somit die Füllung in den Ionisationskammern 8, 22, 23, 24 mit Zählgas überwacht und konstantgehalten bzw. rechnerisch bei der Auswertung berücksichtigt werden.

## Patentansprüche

1. Ionisationskammer für Ionenstrahlen (1) mit einem Kammergehäuse (2), einem Strahleinlaßfenster (3), einem mit Zählgas gefüllten Kammervolumen (5), einer Hochspannungsanode (6) und einer Hochspannungskathode (7), wobei
die Ionisationskammer (8) sandwichartig aus flachen großflächigen Strukturen der obigen Komponenten, die orthogonal zur Ionenstrahlachse ausgerichtet sind, aufgebaut ist, wobei eine zentral angeordnete großflächige orthogonal ausgerichtete flache Zählanode (9) von einer großflächigen flachen Hochspannungskathode (7) aus zwei parallelen Kathodenflächen (10) beidseitig umgeben ist und das Kammergehäuse (2) aus einem Gehäuserahmen (11) besteht, der ein Ionisationskammervolumen (12) nahezu quadratisch umrahmt,
**dadurch gekennzeichnet,**
**daß** ein Strahleinlaßfenster (3) und ein Strahlauslaßfenster (4) in orthogonaler Ausrichtung zum Ionenstrahl (1) gasdicht und elektrisch leitend auf dem Gehäuserahmen (11) angebracht sind, wobei das Strahleinlaßfenster (3) und das Strahlauslaßfenster (4) zu dem Ionisationskammervolumen (12) hin metallisiert sind und wobei die Zählanode (9) und die Hochspannungskathode (7) aus einem metallisch beschichtete Kunststoffasern aufweisenden Gewebe bestehen und in Rahmen (20) eingespannt sind, die sich elektrisch isoliert an dem Gehäuserahmen (11) abstützen, wobei elektrisch isolierende Abstandselemente (21) den Abstand zwischen der Zählanode (9) und der Hochspannungskathode (7) definieren.

2. Ionisationskammer nach Anspruch 1, **dadurch gekennzeichnet, daß** das Zählgas aus einem Gasgemisch aus Argon oder Krypton und Kohlendioxid, vorzugsweise in einem Gaskammervolumen-Mischverhältnis von 4:1 besteht.

3. Ionisationskammer nach Anspruch 1 oder 2, **dadurch gekennzeichnet, daß** der Gehäuserahmen (11) eine selbstdichtende Zählgaseinlaßöffnung (13) und eine selbstdichtende Zählgasablaßöffnung (14) aufweist.

4. Ionisationskammer nach einem der vorhergehenden Ansprüche, **dadurch gekennzeichnet, daß** die Ionisationskammer (8) Sensoren (15, 16), die in gasdicht abgeschlossenen Öffnungen (17) im Gehäuserahmen (11) montiert sind, für den Zählgasdruck (15) und/oder die Zählgastemperatur (16) und/oder die Hochspannung (39) aufweist.

5. Ionisationskammer nach einem der vorhergehenden Ansprüche, **dadurch gekennzeichnet, daß** das Strahleinlaßfenster (3) und das Strahlauslaßfenster (4) aus strahlungbeständigen, nicht polarisierten Kunststoffolien bestehen, die an metallischen, plattenförmigen Rahmen (18) befestigt sind, welche ihrerseits mittels O-Ring-Dichtungen (19) im Gehäuserahmen (11) das Ionisationskammervolumen gasdicht zu dem Strahleinlaßfenster (3) und dem Strahlauslaßfenster (4) abschließen.

6. Ionisationskammer nach einem der vorhergehenden Ansprüche, **dadurch gekennzeichnet, daß** das Strahleinlaßfenster (3) und das Strahlauslaßfenster (4) Polyimid- oder Polyesterfolien aufweisen.

7. Ionisationskammer nach einem der vorhergehenden Ansprüche, **dadurch gekennzeichnet, daß** das Strahleinlaßfenster (3) und das Strahlauslaßfenster (4) zum Ionisationskammervolumen (12) hin aluminiert oder mit Nickel plattiert sind.

8. Ionisationskammer nach einem der vorhergehenden Ansprüche, **dadurch gekennzeichnet, daß** die Zählanode (9) und die Hochspannungskathode (7) aus nickelbeschichtetem Kunststoffgewebe, vorzugsweise nickelbeschichtetem Polyestergewebe, bestehen.

9. Ionisationskammer nach einem der vorhergehenden Ansprüche, **dadurch gekennzeichnet, daß** über Öffnungen (13, 14) im Gehäuserahmen das Zählgas im untersten Bereich in bezug auf die Schwerkraft des Ionisationskammervolumens zuführbar und im obersten Bereich abführbar ist.

10. Ionisationskammer nach einem der vorhergehenden Ansprüche, **dadurch gekennzeichnet, daß** ein Gasdurchflußsensor zur Überwachung des Zählgasdurchflusses außerhalb des Ionisationskammervolumens (12) angeordnet ist.

11. Ionisationskammer nach einem der vorhergehenden Ansprüche, **dadurch gekennzeichnet, daß** die Ionisationskammer (1) bei einem_Abstand von 5 mm zwischen der Hochspannungskathode (7) und der zentralen Zählanode (9) mit einer Hochspannung über 1.500 V aufgrund des Abstandes zwischen Hochspannungskathode (7) und der zentralen Zählanode (9) sowie der Zusammensetzung des Zählgases aus einem Argon/Kohlendioxid- oder Krypton/Kohlendioxid-Gemisch im Verhältnis 80 : 20 % betreibbar ist.

12. Ionisationskammersystem für Ionenstrahlen aus mehreren Ionisationskammern (22, 23, 24) nach einem der vorhergehenden Ansprüche, **dadurch gekennzeichnet, daß** das System zur Intensitätsüberwachung eines Schwerionenstrahls anwendbar ist, wobei mindestens zwei Ionisationskammern (22, 24) in Ionenstrahlrichtung in Reihe hintereinander zur Überwachung der Einzeldosis eines Volumenelements und der Schichtdosis einer gescannten Schicht, sowie zur Überwachung einer Gesamtdosis eines Behandlungszyklus unabhängig von der Ionisationskammer zur Überwachung der Einzeldosis und der Schichtdosis angeordnet sind.

13. Vorrichtung zur Intensitätsüberwachung eines Schwerionentherapiestrahls, mit Ionisationskammern nach einem der vorhergehenden Ansprüche, mit:
a) Mitteln zum Messen der Intensitätsdosis eines Bestrahlungsvolumenelementes eines geplanten Bestrahlungsrasters für eine Bestrahlungsschicht mittels einer ersten Ionisationskammer (22),
b) Mitteln zum Überwachen des gemessenen Wertes der Intensitätsdosis eines Bestrahlungsvolumenelementes durch eine zur ersten Ionisationskammer in Reihe hintereinander angeordnete zweite Ionisationskammer (23),
c) Mitteln zum Vergleichen des Meßwertes der ersten Ionisationskammer mit dem Überwachungswert der zweiten Ionisationskammer und Freigabe zur Bestrahlung des nächsten Bestrahlungsvolumenelementes eines geplanten Bestrahlungsrasters einer Bestrahlungsschicht bei Übereinstimmung mit einer vorgegebenen Sollwertsbreite der Bestrahlungsintensität beider Werte,
d) Mitteln zur Notabschaltung der Bestrahlungsbehandlung bei Überschreiten der vorgegebenen Sollwertsbreite und Nachregeln der Intensität bei Unterschreiten der vorgegebenen Sollwertsbreite,
e) Mitteln zum Wiederholen der Schritte für die nächstfolgenden geplanten Behandlungsschichten, bis das zu bestrahlende Gewebevolumen abgerastert ist,
f) Mitteln zum Integrieren aller gemessenen Bestrahlungsdosen der Überwachungswerte in einer dritten Ionisationskammer (24), die in Reihe hintereinander zu der ersten und zweiten Ionisationskammer angeordnet ist zur Überwachung der Gesamtstrahlungsbelastung eines Gewebevolumens während eines Behandlungszyklus.

14. Vorrichtung nach Anspruch 13, **dadurch gekennzeichnet, daß** die Überwachungsfunktion der ersten (22) und der zweiten Ionisationskammer (23) von einer einzigen Ionisationskammer wahrgenommen wird.

15. Vorrichtung nach einem der Ansprüche 13 oder 14, **dadurch gekennzeichnet, daß** die Vorrichtung zur Überwachung von Ionenintensitäten für Ionenstrahlen mit Ionen schwerer als Protonen ausgebildet ist.

## Claims

1. Ionisation chamber for ion beams (1) having a chamber housing (2), a beam inlet window (3), a chamber volume (5) filled with counting gas, a high-voltage anode (6) and a high-voltage cathode (7) wherein
the ionisation chamber (8) is constructed sandwich-like from flat large-surface-area structures of the above-mentioned components, which are aligned orthogonally relative to the axis of the ion beam, wherein a centrally arranged large-surface-area orthogonally aligned flat counting anode (9) is surrounded on both sides by a large-surface-area flat high-voltage cathode (7) consisting of two parallel cathode surfaces (10), and the chamber housing (2) consists of a housing frame (11) which frames a ionisation chamber volume (12) in a virtually square manner,
**characterised in that**
a beam inlet window (3) and a beam outlet window (4) are mounted gas-impermeably and electrically conductively on the housing frame (11) in orthogonal alignment with the ion beam (1), the beam inlet window (3) and the beam outlet window (4) being metallised on the side facing the ionisation chamber volume (12) and the counting anode (9) and the high-voltage cathode (7) consisting of mesh comprising metal-coated plastics fibres and being mounted in frames (20) which are supported against the housing frame (11) in an electrically insulated manner, with electrically insulating spacing elements (21) defining the spacing between the counting anode (9) and the high-voltage cathode (7).

2. Ionisation chamber according to claim 1, **characterised in that** the counting gas consists of a gas mixture of argon or krypton and carbon dioxide, preferably in a gas chamber volume mixing ratio of 4:1.

3. Ionisation chamber according to claim 1 or claim 2, **characterised in that** the housing frame (11) has a self-sealing counting gas inlet opening (13) and a self-sealing counting gas outlet opening (14).

4. Ionisation chamber according to any one of the preceding claims, **characterised in that** the ionisation chamber (8) has sensors (15, 16) for the counting gas pressure (15) and/or the counting gas temperature (16) and/or the high voltage (39), which sensors are mounted in the housing frame (11) in gas-impermeably sealed openings (17).

5. Ionisation chamber according to any one of the preceding claims, **characterised in that** the beam inlet window (3) and the beam outlet window (4) consist of radiation-resistant non-polarised plastics films, which are secured to metal plate-shaped frames (18), which in turn seal off the ionisation chamber volume gas-impermeably from the beam inlet window (3) and from the beam outlet window (4) by means of O-ring seals (19) in the housing frame (11).

6. Ionisation chamber according to any one of the preceding claims, **characterised in that** the beam inlet window (3) and the beam outlet window (4) comprise polyimide or polyester films.

7. Ionisation chamber according to any one of the preceding claims, **characterised in that** the beam inlet window (3) and the beam outlet window (4) are aluminised or nickel-plated on the side facing the ionisation chamber volume (12).

8. Ionisation chamber according to any one of the preceding claims, **characterised in that** the counting anode (9) and the high-voltage cathode (7) consist of nickel-coated plastics mesh, preferably nickel-coated polyester mesh.

9. Ionisation chamber according to any one of the preceding claims, **characterised in that**, by way of openings (13, 14) in the housing frame, the counting gas can be supplied into the region of the ionisation chamber volume that is lowermost with respect to gravity and can be discharged in the uppermost region.

10. Ionisation chamber according to any one of the preceding claims, **characterised in that** a gas flow sensor for monitoring the throughflow of counting gas is arranged outside the ionisation chamber volume (12).

11. Ionisation chamber according to any one of the preceding claims, **characterised in that** the ionisation chamber (1) can be operated, with a spacing of 5 mm between the high-voltage cathode (7) and the central counting anode (9), at a high voltage of more than 1500 V because of the spacing between the high-voltage cathode (7) and the central counting anode (9) and the composition of the counting gas of an argon/carbon dioxide or krypton/carbon dioxide mixture in a ratio of 80:20 %.

12. Ionisation chamber system for ion beams consisting of a plurality of ionisation chambers (22, 23, 24) according to any one of the preceding claims, **characterised in that** the system can be used to monitor the intensity of a heavy ion beam, with at least two ionisation chambers (22, 24) being arranged in tandem behind one another in the direction of the ion beam in order to monitor the individual dose of a volume element and the layer dose of a scanned layer, and to monitor the total dose of a treatment cycle independently of the ionisation chamber for monitoring the individual dose and the layer dose.

13. Apparatus for monitoring the intensity of a heavy ion therapy beam comprising ionisation chambers according to any one of the preceding claims, comprising:
a) means for measurement of the intensity dose of an irradiation volume element of a planned irradiation raster for an irradiation layer by means of a first ionisation chamber (22);
b) means for monitoring of the measured value of the intensity dose of an irradiation volume element by a second ionisation chamber (23) arranged in tandem behind the first ionisation chamber;
c) means for comparison of the measured value of the first ionisation chamber with the monitoring value of the second ionisation chamber and clearance for the irradiation of the next irradiation volume element of a planned irradiation raster of an irradiation layer when the two irradiation intensity values match within a predetermined desired value range;
d) means for emergency switch-off of the radiation treatment when the predetermined desired value range is exceeded and readjustment of the intensity when it falls short of the predetermined desired value range;
e) means for repetition of the steps for the subsequent planned treatment layers until the tissue volume to be irradiated has been fully scanned;
f) means for integration of all the measured radiation doses of the monitoring values in a third ionisation chamber (24), which is arranged in tandem behind the first and second ionisation chambers, in order to monitor the total radiation to which a tissue volume is subjected during a treatment cycle.

14. Apparatus according to claim 13, **characterised in that** the monitoring function of the first ionisation chamber (22) and of the second ionisation chamber (23) is provided by a single ionisation chamber.

15. Apparatus according to any one of claims 13 or 14, **characterised in that** the apparatus is formed to monitor ion intensities for ion beams with ions that are heavier than protons.

## Revendications

1. Chambre d'ionisation pour faisceaux ioniques (1) comprenant un logement de chambre (2), une fenêtre d'entrée du faisceau (3), un volume de chambre rempli de gaz de comptage (5), une anode sous haute tension (6) et une cathode sous haute tension (7), dans laquelle la chambre d'ionisation (8) est constituée d'un empilement des structures plates de grande taille des composants ci-dessus, dirigés orthogonalement vers l'axe du faisceau ionique, dans laquelle la grande anode de comptage plate dirigée orthogonalement et disposée de manière centrale (9) d'une cathode sous haute tension plate de grande taille (7) est entourée de deux surfaces cathodiques parallèles (10) des deux côtés et le logement de chambre (2) est constitué d'un cadre de logement (11), qui englobe un volume de la chambre d'ionisation presque carré (12), **caractérisée en ce que** sur le cadre de logement (11) sont placées une fenêtre d'entrée du faisceau (3) et une fenêtre de sortie du faisceau (4) dans une direction orthogonale au faisceau ionique (1) conduisant l'électricité et le gaz sur le cadre de logement (11), où la fenêtre d'entrée du faisceau (3) et la fenêtre de sortie du faisceau (4) sont métallisées sur le volume de la chambre d'ionisation (12) et où l'anode de comptage (9) et la cathode sous haute tension (7) sont constituées d'un tissu revêtu de métal présentant des fibres de plastique et sont fixées dans des cadres (20), qui s'appuient de manière électriquement isolée dans le cadre de logement (11), où les éléments d'écart électriquement isolants (21) définissent l'écart entre l'anode de comptage (9) et la cathode sous haute tension (7).

2. Chambre d'ionisation selon la revendication 1, **caractérisée en ce que** le gaz de comptage est constitué d'un mélange de gaz d'argon ou de krypton et de dioxyde de carbone, de préférence en un rapport de mélange du volume de la chambre de 4:1.

3. Chambre d'ionisation selon la revendication 1 ou 2, **caractérisée en ce que** le cadre de logement (11) comporte une ouverture d'entrée du gaz auto-étanche (13) et une ouverture de sortie du gaz de comptage auto-étanche (14).

4. Chambre d'ionisation selon l'une quelconque des revendications précédentes, **caractérisée en ce que** la chambre d'ionisation (8) comporte des capteurs (15, 16) montés dans les ouvertures fermées hermétiques au gaz (17) dans le cadre de logement (11), pour la pression du gaz de comptage (15) et/ou la température du gaz de comptage (16) et/ou la haute tension (39).

5. Chambre d'ionisation selon l'une quelconque des revendications précédentes, **caractérisée en ce que** la fenêtre d'entrée du faisceau (3) et la fenêtre de sortie du faisceau (4) sont constituées de feuilles de plastique résistantes au faisceau, non polarisées, fixées sur un cadre métallique de forme plate (18), lesquelles ferment quant à elles au moyen de joints toriques (19) dans le cadre de logement (11) le volume de la chambre d'ionisation de manière hermétique au gaz à la fenêtre d'entrée du faisceau (3) et la fenêtre de sortie du faisceau (4).

6. Chambre d'ionisation selon l'une quelconque des revendications précédentes, **caractérisée en ce que** la fenêtre d'entrée du faisceau (3) et la fenêtre de sortie du faisceau (4) comportent des feuilles de polyimide ou de polyester.

7. Chambre d'ionisation selon l'une quelconque des revendications précédentes, **caractérisée en ce que** la fenêtre d'entrée du faisceau (3) et la fenêtre de sortie du faisceau (4) sont aluminées ou plaquées en nickel sur le volume de la chambre d'ionisation (12).

8. Chambre d'ionisation selon l'une quelconque des revendications précédentes, **caractérisée en ce que** l'anode de comptage (9) et la cathode sous haute tension (7) sont constituées d'un tissu plastique revêtu de nickel, de préférence un tissu de polyester revêtu de nickel.

9. Chambre d'ionisation selon l'une quelconque des revendications précédentes, **caractérisée en ce que** par les ouvertures (13, 14) dans le cadre de logement, le gaz de comptage peut être amené dans la zone la plus basse du point de vue de la gravitation du volume de la chambre d'ionisation et peut être évacué de la zone la plus élevée.

10. Chambre d'ionisation selon l'une quelconque des revendications précédentes, **caractérisée en ce qu'**un capteur du débit de gaz pour le contrôle du débit de gaz de comptage est disposé à l'extérieur du volume de la chambre d'ionisation (12).

11. Chambre d'ionisation selon l'une quelconque des revendications précédentes, **caractérisée en ce que** la chambre d'ionisation (1) est exploitable avec une distance de 5 mm entre la cathode sous haute tension (7) et l'anode de comptage centrale (9), avec une haute tension de plus 1 500 V en raison de l'écart entre la cathode sous haute tension (7) et l'anode de comptage centrale (9) ainsi que de la composition du gaz de comptage à partir d'un mélange argon/dioxyde de carbone ou krypton/dioxyde de carbone à un rapport 80:20 %.

12. Système de chambre d'ionisation pour faisceaux d'ions de plusieurs chambres d'ionisation (22, 23, 24) selon l'une quelconque des revendications précédentes, **caractérisé en ce que** le système peut être employé pour le contrôle d'intensité d'un faisceau d'ions lourds, où au moins deux chambres d'ionisation (22, 24) sont disposées dans la direction du faisceau d'ions en ligne l'une derrière l'autre pour le contrôle de la dose unique d'un élément de volume et de la dose par couche d'une couche scannée, ainsi que pour le contrôle de la dose totale d'un cycle de traitement indépendamment de la chambre d'ionisation pour le contrôle de la dose unique et de la dose par couche.

13. Dispositif de contrôle de l'intensité d'un faisceau de traitement par ions lourds au moyen de chambres d'ionisation selon l'une quelconque des revendications précédentes comprenant :
a) des moyens de mesure du dosage d'intensité d'un élément de volume de rayonnement d'une trame de rayonnement prévue pour une couche de rayonnement au moyen d'une première chambre d'ionisation (22),
b) des moyens de contrôle de la valeur mesurée du dosage d'intensité d'un élément de volume de rayonnement par une deuxième chambre d'ionisation (23) disposée dans un alignement derrière la première chambre d'ionisation,
c) des moyens de comparaison de la valeur mesurée de la première chambre d'ionisation avec la valeur de contrôle de la deuxième chambre d'ionisation et de libération de rayonnement de l'élément de volume de rayonnement suivant d'une trame de rayonnement prévue d'une couche de rayonnement par conformité avec une plage de valeurs de consigne prédeterminée de l'intensité de rayonnement des deux valeurs,
d) des moyens de déconnexion d'urgence du traitement par rayonnement en cas de dépassement de la plage de valeurs de consigne et de reréglage de l'intensité en cas de passage sous le seuil de la plage de valeurs de consigne prédéterminée,
e) des moyens de répétitions des étapes pour les couches de traitement prévues consécutivement, jusqu'à ce que le volume de tissu à irradier soit couvert,
f) des moyens d'intégration de tous les dosages de rayonnement mesurés des valeurs de contrôle dans une troisième chambre d'ionisation (24), disposée dans un alignement derrière la première et la deuxième chambres d'ionisation, pour le contrôle de la charge de rayonnement totale d'un volume de tissu au cours d'un cycle de traitement.

14. Dispositif selon la revendication 13, **caractérisé en ce que** la fonction de contrôle de la première (22) et la deuxième (23) chambres d'ionisation est assurée par une chambre d'ionisation unique.

15. Dispositif selon la revendication 13 ou 14, **caractérisé en ce que** le dispositif de contrôle de l'intensité des ions est conçu pour des faisceaux d'ions avec des ions plus lourds que des protons.
